Europäisches Patentamt

European Patent Office

Office européen des brevets

(19)

(11) Publication number: 0 072 257
A2

# (12) EUROPEAN PATENT APPLICATION

(21) Application number: 82304247.8

(22) Date of filing: 11.08.82

(51) Int. Cl.³: **A 61 L 2/18**, A 61 L 2/24, B 08 B 3/08

(30) Priority: 12.08.81 GB 8124658

(43) Date of publication of application: 16.02.83 Bulletin 83/7

(84) Designated Contracting States: **AT BE CH DE FR GB IT LI LU NL SE**

(71) Applicant: **KEYMED (MEDICAL & INDUSTRIAL EQUIPMENT) LIMITED, Keymed House Stock Road, Southend-on-Sea SS2 5QH (GB)**

(72) Inventor: **Gray, Roger Leslie, 4 Brookside Hawkwell, Hockley Sussex SS5 4EW (GB)**
Inventor: **Parker, George Christopher, 40 Henley Crescent, Westcliff-on-Sea Essex SS0 9NT (GB)**
Inventor: **Arnold, Allan Peter, 64 Colville CLose, Corringham Essex (GB)**

(74) Representative: **Hardisty, David Robert et al, BOULT, WADE & TENNANT 27 Furnival street, London EC4A IPQ (GB)**

(54) Fibrescope cleaning and disinfection apparatus.

(57) Apparatus for disinfecting a flexible endoscope including a container 9 for disinfectant solution, a container 10 for rinsing solution, and means for passing the disinfectant solution and the rinsing solution in turn through all of the channels of the flexible limb of the endoscope, and thence to waste. The solutions are used only once. The apparatus includes a removable cradle 30 for holding the body portion of various types of endoscope. A latch 42 is provided to hold the body portion 39 in position on the cradle 30, and a safety interlock prevents operation when the body portion is not latched into position.

The apparatus includes means 33 for automatically operating valves onto the body portion of the endoscope to control flow of fluid in the endoscope channels.

# FIBRESCOPE CLEANING AND DISINFECTION APPARATUS

This invention relates to apparatus, e.g. an automatic machine, for washing, cleaning and disinfecting the internal channels of flexible fibreoptic endoscopes (or 'fibrescopes') by a method of non-recirculatory irrigation.

Flexible endoscopes are used for a variety of medical purposes, for example in the examination and treatment of the gastro-intestinal and respiratory tracts. They generally comprise a flexible limb including an image guide, a light guide, a working channel (sometimes known as the biopsy channel) and an auxiliary channel. The working channel is used for the passage of various surgical instruments and the like to the distal end of the endoscope and for the aspiration of various body fluids and the auxiliary channel is normally connected to water and air supplies, to enable organ insufflation, and cleaning of a viewing window at the distal end of the endoscope when the instrument is in use. In a number of instruments which are presently available commercially the air and water supplies to the auxiliary channel join the auxiliary channel, and each other at a point along the flexible limb.

A body portion is attached to the flexible limb, and includes means defining an aperture through which viewing may take place. This will usually include an eyepiece, but may be, for example, a mount for a television camera.

Channels are normally provided to the body portion for supplying air and/or water to the auxiliary channel, often via separate channels through the flexible limb and for supplying suction to the working channel, when required, so that the operator of the instrument is able to maintain a clear field of vision at all times. These channels are often enclosed in a second flexible limb, or so-called "umbilical cord" together with the light guide for directing light to the proximal end of the instrument.

Fibrescopes are, by their nature, difficult instruments to clean and disinfect internally, because of the various channels in which unwanted contents of the gastro-intestinal and respiratory tracts may be aspirated.

To keep the likelihood of cross-infection to a minimum, thorough cleaning and disinfection procedures are recommended, but using present methods, some internal parts of the fibrescope still remain relatively "dirty" because some of the methods recommended for cleaning and disinfecting involve complicated procedures requiring at least two trained personnel, and take up more time than is generally available between examinations in hospital endoscopy departments.

There are currently available a number of automatic machines for the cleaning and disinfection of flexible endoscopes, but none of those available clean all of the channels that are most prone to blockage namely the channels (including the auxiliary channel) for passing air and water through the flexible limb. Any channel which, in use, passes only air will be particularly prone to blockage, as it will not normally be irrigated either during use or cleaning procedures and the instruments referred to above in which separate air and water supplies join the auxiliary channel at a point along the length of the flexible limb are particularly prone to blockage at the junctions. Serious blockage of the air channel requires the fibrescope to be returned to a specialised service centre for attention by skilled technicians, which is a lengthy and expensive procedure.

Most of the existing machines devote a large amount of effort to cleaning and disinfecting the outer sheath of the flexible limb (or "patient tube") of the fibrescope, which can be cleaned and disinfected quickly and adequately by hand. Furthermore, existing machines generally operate by recirculating the cleaning and disinfection fluids, sometimes without filtration, so that by the end of an endoscopy list (usually between four and ten patients) the fibrescope is being washed with contaminated detergent and disinfectant.

According to the invention there is provided apparatus for disinfecting a flexible endoscope having a flexible limb including an image guide, a light guide, a working channel, and at least one auxiliary channel for passing air and water through the flexible limb, a body portion attached to the flexible limb, at least two supply channels for supplying suction, and air and water respectively to the body portion, the apparatus including a container for a disinfectant liquid, a container for a rinsing liquid and comprising means for placing both the container for disinfectant liquid and the container for rinsing liquid together in valved communication with both the said channels of the flexible limb and means for passing the disinfectant liquid and the rinsing liquid in turn through all of the channels of the flexible limb and for passing the used liquids to waste, whereby the said liquids are used only once.

In an alternative embodiment the invention provides apparatus for disinfecting a flexible endoscope having a flexible limb including an image guide, a light guide, a working channel and an auxiliary channel, a body portion attached to the flexible limb, and channels for supplying suction, air and water to the body portion the apparatus including a container for a disinfectant liquid, a container for a rinsing liquid and means for passing the disinfectant liquid and the rinsing liquid in turn through all of the channels of the flexible limb, a cradle for holding the body portion, a latch to hold the body portion in position on the cradle, and means for preventing the flow of the said liquids when the body portion of an endoscope is not latched into the cradle. The cradle for the endoscope body may preferably be made removable, and a series of such cradles may be provided for the machine, to accommodate different endoscope designs.

Most designs of endoscope in common use include at least one, and normally two, manually operable valves on the body portion of the endoscope, to control the flow of air and water into the flexible limb and to control the suction applied at the distal end through the flexible limb.

In order to provide efficient cleaning of all the internal passageways of the instrument, we believe it advisable to operate these valves during the disinfection cycle.

Accordingly, in yet a further embodiment of the invention, there is provided apparatus for disinfecting a flexible endoscope including means for automatically operating at least one valve on the body portion of the endoscope, to control the flow of fluid in the channels of the endoscope during the disinfection cycle.

As indicated above, the apparatus of the invention includes a container for a rinsing liquid, and a container for a disinfectant liquid. In addition, a third container for detergent liquid may optionally be provided, although it may be sufficient in certain circumstances to utilise a single container containing a mixture of disinfectant and detergent, in conjunction with a rinsing liquid container.

In a preferred embodiment, the apparatus includes a control circuit to provide a minimum time period for the disinfectant and/or detergent part of the apparatus cycle. This minimum time period is preset to be the minimum period thought necessary for disinfection of the instrument, and this part of the cycle may not be shortened manually. An override may however be provided to allow the user the option of a longer disinfectant or detergent part of the cycle.

A preferred embodiment of the invention will now be described with reference to the accompanying drawings, in which:-

Figure 1 is a side elevation of apparatus according to the invention, with a flexible endoscope in position for disinfection,

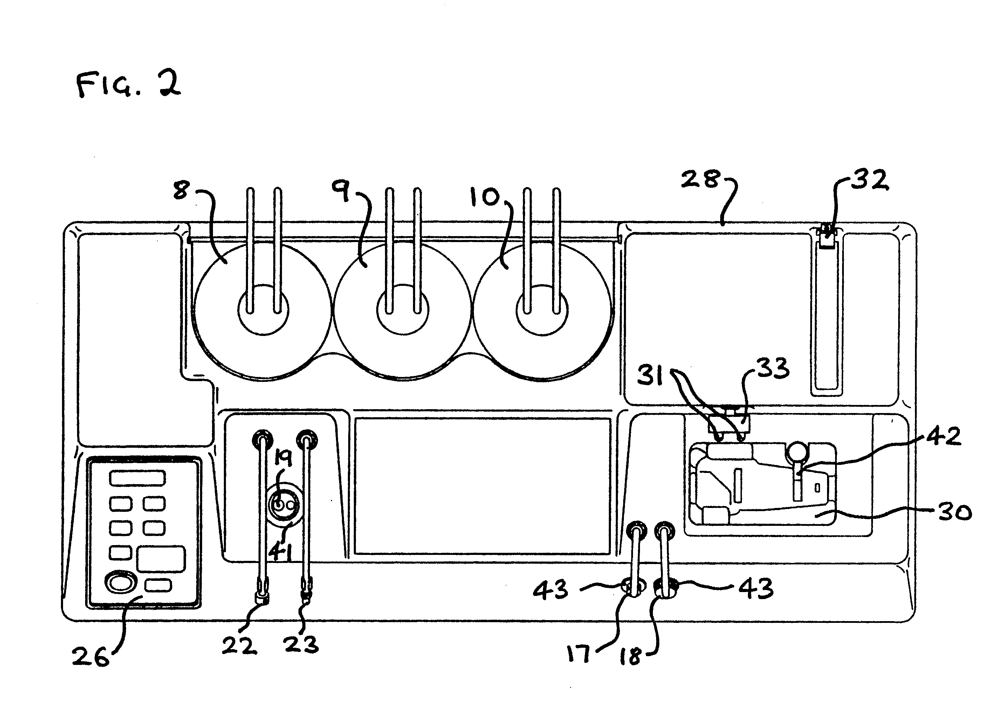

Figure 2 is a plan view of the machine of Figure 1, with the endoscope removed, and

Figure 3 is a schematic pneumatic/hydraulic circuit diagram of the apparatus of Figures 1 and 2.

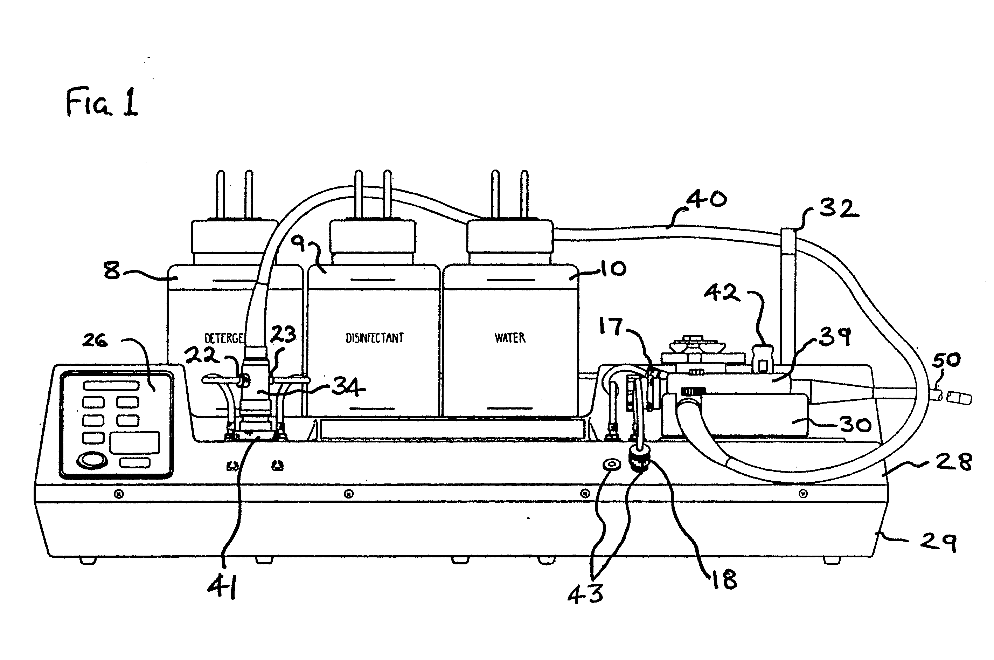

As shown in Figure 1, the apparatus illustrated comprises a base housing comprising a bottom cover 29 and a top cover 28 which together form a housing for the control mechanisms and machinery of the apparatus whilst providing a tray-like top surface.

Bottles for detergent (8), disinfectant (9) and water (10) stand in a small depression in the tray-like top designed to prevent them from sliding if the apparatus is tilted slightly and to indicate to an operator where the bottles should stand.

Each bottle has an air-tight top through which two pipes enter the bottle. One pipe in each case extends into the liquid in the bottle and the other terminates above the liquid level. Each of these shorter pipes connects to an air pump via a conduit 2 contained within the base housing by which each bottle may be pressurised to drive liquid out through the respective pipe extending into the liquid.

A cradle 30 is provided on the tray-like top to receive the body portion of an endosccpe to be cleaned. The cradle is shaped to support a particular type of endoscope and is accordingly made removable so that alternative shapes of cradle can be inserted to accommodate other designs of endoscope.

A cross-latch 42 is provided to monitor the presence of an endoscope body in the cradle. The cross-latch 42 is pivoted into position on top of the fibrescope control body once the control body is correctly positioned in the cradle 30. Electronic circuitry including an electro-mechanical interlock is provided to prevent the apparatus operating if cross-latch 42 fails to indicate the presence of an endoscope control body in the cradle.

The endoscope to be cleaned will generally have one, or more usually two, piston valves on its control body. Generally one piston valve having a hole in the upper part thereof which, unless covered, allows the air channel to be vented to atmosphere. When the hole is covered, air passes from the air channel through the auxiliary channel of the instrument, and when the valve is pressed water flows into the auxiliary channel.

Generally the second piston valve controls the application of suction to the working channel and opens communication between the suction channel of the umbilical cord and the working channel of the flexible limb when depressed.

In order to operate these piston valves during the cleaning process a pair of buttons 31 are provided on a metal frame 31. The button/frame assembly is made so as to be easily changeable by hand to deal with different endoscope designs. The frame is mounted for reciprocating movement by the action of a geared electric motor driving a snail-cam within the machine casing, there being a cam-follower therefor attached to the button frame 33.

In order to avoid leakage of the washing fluids, the buttons 31 which cover and depress the fibrescope air/water and suction pistons are preferably manufactured from silicone rubber.

A post 32 is provided standing up from the back of the tray-like top to support a portion of the umbilical cord 40 of the endoscope for convenience and safety. A suitable clip is provided at the top of post 32 for gripping the umbilical cord without damaging it.

At the extreme left front of the top cover 28 is a control panel 26 containing switches for controlling the operation of the apparatus as will be described hereafter.

Adjacent the switch panel toward the front of the tray-like top of the apparatus is a connector 41 set in the top. Connector 41 is designed to connect to the fitting at the end of the umbilical cord 40 of the endoscope and contains a connector 19 for connecting to the air channel of the endoscope. Connector 41 also comprises means for sensing the presence of the connector of the umbilical cord. The sensor is connected to an electro-mechanical interlock to prevent the machine being activated when this part of the fibrescope is not properly positioned.

To the rear of connector 41 a pair of pipes emerge from the top cover and terminate in connectors 22 and 23 for connection to the air channel and water channel connectors of the umbilical cord of the endoscope.

Adjacent and to the left of the cradle 30 a further pair of pipes emerge from the top and terminate in connectors 17 and 18. These connectors are designed to connect to the working channel or channels of the endoscope at the control body. A blind part into which either of connectors 17, 18 can be fitted sealingly is provided on the top cover so that one of the two pipes can be plugged when the endoscope to be cleaned contains only one working channel.

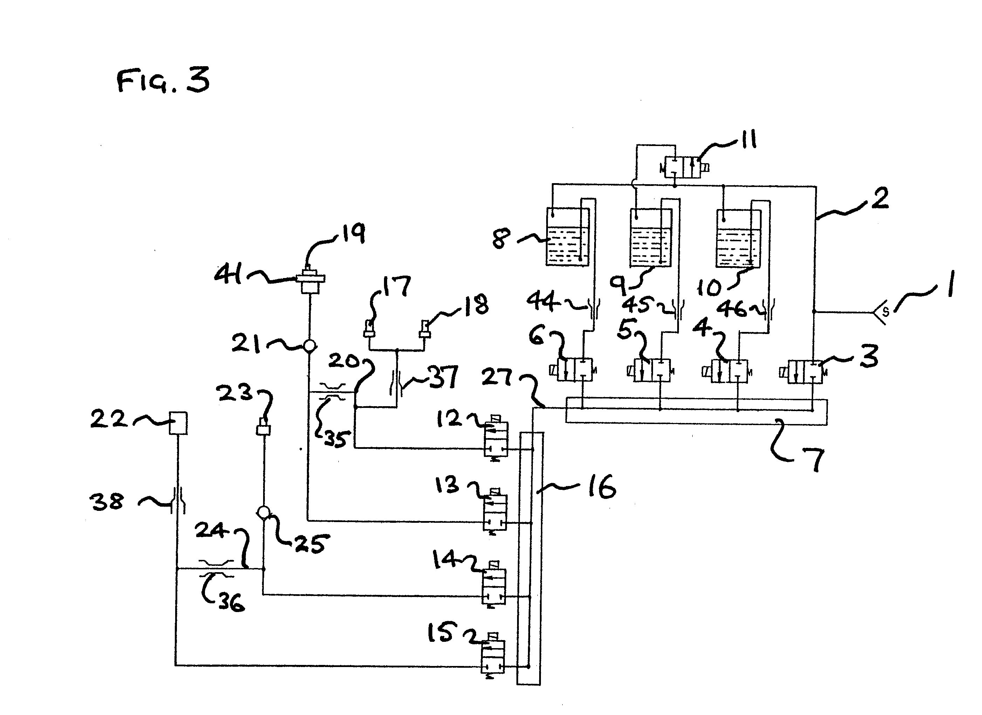

Inside the housing the components described above are connected as shown in Figure 3.

Air pump 1 is connected to the air space in each bottle 8, 9, 10. The pump is in permanent communication with bottles 8 and 10 for detergent and rinsing water respectively but is in communication which bottle 9 for disinfectant only via a solenoid operated shut-off valve 11.

Each bottle is in communication for liquid flow with a manifold 7 via a respective solenoid operated shut-off valve 4, 5 or 6 by a pipe or conduit which dips below the liquid level in bottle and contains a flow restrictor 44, 45 or 46.

Manifold 7 is in communication for liquid flow with a second manifold 16 via a pipe or conduit 27.

Four pipes leave manifold 16 and each contains a solenoid operated valve 12, 13, 14 or 15.

A first of the four pipes leads via a flow restrictor 37 to a branch point where it splits into two branch pipes which are the two pipes that exit through the top cover adjacent the cradle and terminate in connectors 17 and 18 for connection to the working channel(s).

The second of the four pipes leads to a non-return valve 21 and thence to connector 19 (in the larger connector 41) to which the air channel of the endoscope connects.

Upstream of non-return valve 21, the second pipe is connected to the first pipe by a flow restrictor 35.

The third of the four pipes leads through a non-return valve 25 out of the top cover to connector 23 for the water channel.

The last of the four pipes leads through a flow restrictor 38 out of the top cover to connector 22 for the suction channel. Upstream of restrictor 38 a flow restrictor 36 links the fourth pipe to the third.

Electrical circuitry is provided to connect the control panel 26, the air pump 1, the solenoid operated valves 3 to 6 and 11 to 15, and the electric motor driving frame 33 and to allow operation of these in programmed sequence as described below.

All the control circuitry, both pneumatic/hydraulic and electronic, is housed inside the covers 28, 29 and the only external connection required is that of mains electricity.

The electric circuitry also connects the cross-latch 42 and the sensor for the presence of a light guide connector in connector 41 so as to prevent operation of the apparatus unless an endoscope is present.

In use an endoscope is positioned with its control body in the cradle 30 and cross-latch 42 is moved to engage and sense the control body.

The flexible limb of the endoscope is led to a suitable waste liquid container or drain and the umbilical cord is supported in its mid region on post 32 and connected at its free end to connector 41. The making of this connection is sensed by the sensor in the connector 41.

Connections are made between connector 22 and the endoscope suction channel, connector 23 and the endoscope water channel, and connector 18 and the working channel. If there is a second working channel it is connected to connector 17 otherwise this is closed by connection to blind port 43. The connection of the umbilical cord to connector 41 automatically connects the air channel to connector 19.

The machine illustrated can be run on either a 'two-bottle' or 'three-bottle' cycle this being selected prior to the fibrescope being disinfected. On the 'three-bottle' cycle, the three bottles supplied with the machine are filled with, a detergent solution, a disinfectant solution, and water respectively. On the 'two-bottle' cycle, only two (8),(10) of the three bottles are utilised, and it is recommended that a detergent/disinfectant mixture is used in the first bottle, with water in the other.

The control panel 26 includes "mains on/off" switch; selector switches for "two-bottle" and "three-bottle" operation, "cycle pause", "restart after pause" and "cycle start" switches.

In a preferred embodiment, the control panel includes a timer that shows the time left to complete the selected cycle, and indicator lights to show "machine ready", "cycle in progress", "pause in progress" and "cycle complete".

The use and purpose of these switches will be explained in the following description of the operation of the apparatus.

The mains on/off switch having been moved to the "on" position and the endoscope to be cleaned having been positioned and connected, two or three bottle operation is selected.

The apparatus will then upon operation of the "cycle start" switch commence a cleaning cycle which will be carried on automatically.

The minimum cycle times for each fluid for both two and three-bottle cycles are preferably preset. The preferred times and order of cleaning operations are as follows:

- 13 - 0072257

a) Three-bottle cycle:

30 seconds detergent through biopsy and air channels

30 seconds detergent through water and suction channels

45 seconds disinfectant through water and suction channels

45 seconds disinfectant through biopsy and air channels

60 seconds water through biopsy and air channels

60 seconds water through water and suction channels

45 seconds air through biopsy and air channels

45 seconds air through water and suction channels.

b) Two-bottle cycle:

60 seconds detergent/disinfectant mixture through biopsy and air channels

60 seconds detergent/disinfectant mixture through water and suction channels

60 seconds water through water and suction channels

60 seconds water through biopsy and air channels

45 seconds air through biopsy and air channels

45 seconds air through water and suction channels.

This results in a total cycle time of approximately six minutes for the three-bottle cycle and 5½ minutes for the two-bottle cycle. These cycle times have been shown to be micro-biologically effective when certain recommended detergents and disinfectants are used, as long as the fibrescopes have been cleaned externally, and the biopsy channel(s) brushed through according to the cleaning instructions supplied with each fibrescope prior to connection to the machine. In addition, these cycle times are short enough to enable every fibrescope to be automatically cleaned and disinfected internally by the machine between each patient on an endoscopy list.

If patients are known or suspected carriers of particularly virulent bacteria, such as infectious hepatitis, a built-in 'pause' function enables the machine to be stopped in either the three-bottle or two-bottle cycle with disfectant present in all channels of the fibrescope by operation of the "cycle pause" switch. This can then be left in the channels for as long a period as desired, at the discretion of the local hospital microbiologist. When sufficient time has elapsed, the 'restart' control can be activated, and the machine will then complete the previously designated cycle.

When the "cycle pause" switch has been operated, the machine pauses at a predetermined point during the disinfectant part of the cycle, until the "restart after pause" switch is operated.

The 'pause' control can be operated at any time from the start of the cycle to the actual moment of the cycle pausing. This moment is halfway through the second disinfectant flushing on the three-bottle cycle, and halfway through the second detergent/disinfectant mixture flushing on the two-bottle cycle.

The machine blows air through the channels of the fibrescope after the water rinse cycle, in order to eject the water and dry the channels as mush as possible before being re-used or stored.

During the cycle, the two rubber buttons control the air/water and suction pistons on the side of the control body, and these pistons are depressed and released by the rubber buttons at various times during the cycle, according to the programme selected, in order to let the cleaning and disinfecting fluids have access to all internal channels of the fibrescope.

During operation, air pump 1 pressurises outer bottles 8 and 10 via conduit 2. When solenoid valve 11 is in the closed position as it is when three-bottle operation is selected, the centre bottle 9 is also pressurised.

An electrical circuit (not shown) connects the solenoid valve 11 to control panel 26 of the apparatus, to enable the user to select either two or three-bottle operation. Because, during two-bottle operation, solenoid valve 11 remains closed, bottle 9 does not need to be made airtight during two-bottle operation.

It can be seen that opening any one of the solenoid valves 12, 13, 14 and 15 will lead to fluid being pumped through any one of the previously mentioned connectors 17, 18 (connected together), 19, 22, 23. In a preferred embodiment of this invention, solenoid valves 12 and 13 operate together and solenoid valves 14 and 15 operate together, so that the working channel and air channel and the connected auxiliary channel are irrigated at the same time, and the suction channel and connected working channel and the water channel and connected auxiliary channel are irrigated at the same time.  This is convenient from a practical point of view as, in many designs of endoscope, the air and water channels cannot be flushed at the same time since only one or the other can be open to the auxiliary channel, nor can the biopsy (or working) and suction channels.  This is because the air/water valve on the side of the fibrescope control body 39 must be in a different position in order to access the air channel and the water channel, and similarly, the aspiration piston must be in a different position in order to access the working channel and the suction channel.

The working channel connectors 17, 18 will both be used if a 'twin-channel' fibrescope is to be disinfected, but normally only one would be used, the second connector being plugged into one of the 'blind' ports 43 in the front of the machine, so that fluid does not leak from the machine.

If there is a blockage in the air channel of the flexible limb of the fibrescope, then the irrigation action of the detergent being flushed through the channel should clear it, provided that the fibrescope has only recently been used.  However, if the fibrescope has been left for some time before being connected to the machine, a blockage in the air channel may have dried and hardened.  The flushing action of the machine may therefore not clear the blockage, in which case there may be a static pressure build-up at the air channel connector 19 that might result in a breakdown of a seal in the light guide connector 34 of the fibrescope, causing damage to the instrument. For this reason, link pipe 20 is provided between the air and biopsy channels pipework (the working channel rarely blocks, and may be cleaned by brushing prior to connection to the machine) to detour any fluid intended for the air channel to the working channel should the air channel be blocked. The one-way valve 21 is situated in the pipe joining the connector 19 in the link pipe 20 to prevent backflow from the water connector during irrigation of the water channel. Flow restrictor 35 is included in the link pipe 20 in order to 'balance' the flow through the connectors in normal usage.

Similarly, link pipe 24 joins the pipes to the water and suction channels to allow for blockage in the water channel, and one-way valve 25 within the water connector 23 prevents backflow from the air channel during irrigation of the air channel, together with flow restrictor 36 in the pipe link 24.

The fluid flow is balanced in normal usage by use of flow restrictors 37, 38, 44, 45, 46 in certain runs of the pipework, as the working and suction channels of a fibrescope are normally of a larger diameter than the air, auxiliary and water channels.

The top cover 28 and bottom cover 29 are preferably vacuum-moulded from a high strength plastic material such as ABS, so that the machine is relatively lightweight, and thus fully portable by nursing staff (who are normally responsible for fibrescope cleaning and disinfection).

The bottles 8, 9, 10 of the machine are preferably manufactured from a material than can be autoclaved, such as polypropylene, so that, should any contact with particularly virulent bacteria occur, the bottles can be sterilised.

The bottles 8, 9 and 10 are of approximately 2 litre capacity each, and the overall dimensions of the machine are approximately 800mm x 300mm.

All the pipes of the machine should be capable of being autoclaved, and thus are preferably made from materials such as silicone rubber or polyurethane.

For ease of sterilisation, the pipework external to the machine is easily removable by hand, and is colour-coded for correct replacement.

Cradle 30 in which the fibrescope control body 39 is held is preferably manufactured of a soft but firm rubber, such as room-temperature vulcanising silicone rubber.

All the fluids from the bottles are flushed through the channels of the fibrescope by the action of an air pump pressurising the three bottles, forcing the fluids to flow through a series of solenoid valves controlled in a programmed sequence by a digital electronics control system. This control system ensures that each valve is opened in correct sequence to allow flow of the appropriate fluid to the appropriate channels at the appropriate time. The distal end of the fibrescope is positioned over a sink or other waste outlet, so that the contaminated fluids are disposed of immediately.

In the embodiment described, fluid flow during the cycle is towards the distal end of the instrument.  This has the advantage that if any foreign material is present in any of the channels of the endoscope, they are flushed out of the instrument by the shortest route.

It will of course be appreciated that the apparatus according to the invention may be used with other specific endoscope designs.

CLAIMS

1. Apparatus for disinfecting a flexible endoscope having a flexible limb 50 including an image guide, a light guide, a working channel, and at least one auxiliary channel for passing air and water through the flexible limb, a body portion 39 attached to the flexible limb, at least two supply channels for supplying suction, and air and water respectively to the body portion, the apparatus including a container 9 for a disinfectant liquid, a container 10 for a rinsing liquid and being characterised in that the apparatus comprises means 3, 4, 5, 6, 7, 27, 16, 12, 13, 14, 15 for placing both the container for disinfectant liquid and the container for rinsing liquid together in valved communication with both the said channels of the flexible limb and means for passing the disinfectant liquid and the rinsing liquid in turn through all of the channels of the flexible limb and for passing the used liquids to waste, whereby the said liquids are used only once.

2. Apparatus for disinfecting a flexible endoscope having a flexible limb including an image guide, a light guide, a working channel, and an auxiliary channel, a body portion 39 attached to the flexible limb, at least two supply channels for supplying suction, and air and water respectively to the body portion, the apparatus comprising a container 9 for a disinfectant liquid, a container 10 for a rinsing liquid, and characterised in that the apparatus comprises means for passing disinfectant liquid and the rinsing liquid in turn through all of the channels of the flexible limb, a cradle 30 for holding the body portion, a latch 42 to hold the body portion in position on the cradle, and means 42 preventing the flow of the said liquids when the body portion of an endoscope is not in the cradle.

3. Apparatus as claimed in claim 2 further characterised in that the cradle 30 is removable.

4. Apparatus for disinfecting a flexible endoscope of the kind having a flexible limb including an image guide, a light guide, a working channel, an an auxiliary channel, a body portion 39 attached to the flexible limb, at least two supply channels for supplying suction, and air and water respectively to the body portion, and at least one valve on the body portion controlling fluid access from the supply channels to the channels of the flexible limb, the apparatus including a container 9 for a disinfectant liquid, a container 10 for a rinsing liquid, and being characterised in that the apparatus comprises means for passing the disinfectant liquid and the rinsing liquids in turn through all of the channels of the flexible limb and means 31, 33 for automatically operating the valve or valves on the body portion of the endoscope to control the flow of liquid in the said channel during the disinfection process.

5. Apparatus as claimed in any one of claims 1 to 4, further characterised in that the means for passing the disinfectant liquid through the working and auxiliary channels comprises a tube connected to supply the liquid to the working channel, and a tube connected to supply the liquid to the auxiliary channel, the said tubes being interconnected 20, whereby an excess of pressure in the tube connected to the auxiliary channel may be vented to the working channel.

6. Apparatus as claimed in any one of claims 1 to 5, further characterised in that the means for passing disinfectant and rinsing liquids through the working and auxiliary channels includes a tube for connection to the channel for applying suction to the body portion, and a tube for connection to the channel for supplying air and/or water to the body portion, the said tubes being interconnected 24 whereby an excess of pressure in the channel for supplying air and/or water to the body portion may be vented to the channel for supplying suction.

7. Apparatus as claimed in any one of the preceding claims, further characterised by including a container for a detergent liquid, and means for passing the detergent liquid, the disinfectant liquid, and the rinsing liquid in turn through both the working and auxiliary channels of the flexible limb.

8. Apparatus as claimed in any one of the preceding claims, further characterised in that the said containers 9, 10 are each in valved connection with a common manifold 7, means being provided for connecting the said manifold 7 to the channels of the flexible limb.

9. Apparatus as claimed in any one of the preceding claims, further characterised by including a control circuit connected to step the apparatus through periods of supply of disinfectant and rinsing solution and to provide a minimum time period within which disinfectant is passed through the said working and auxiliary channels.

10. Apparatus as claimed in claim 9, further characterised by including interrupt means operable to increase the period for which disinfectant is passed through the said working and auxiliary channels over minimum time period.

Fig. 1

FIG. 2

Fig. 3

3/3

0072257